(19) Europäisches Patentamt
European Patent Office
Office européen des brevets

(11) Veröffentlichungsnummer : **0 035 697**
**B1**

(12) **EUROPÄISCHE PATENTSCHRIFT**

(45) Veröffentlichungstag der Patentschrift : **15.12.82**

(21) Anmeldenummer : **81101349.9**

(22) Anmeldetag : **25.02.81**

(51) Int. Cl.³ : **C 07 C 43/23**, C 07 C 41/03, C 07 D249/08, C 07 D233/60// A01N43/64, A01N39/00

# (54) 3,3-Dimethyl-1-phenoxy-butan-2-ole, Verfahren zu ihrer Herstellung sowie ihre Verwendung als Zwischenprodukte.

(30) Priorität : **05.03.80 DE 3008477**

(43) Veröffentlichungstag der Anmeldung : **16.09.81 (Patentblatt 81/37)**

(45) Bekanntmachung des Hinweises auf die Patenterteilung : **15.12.82 Patentblatt 82/50**

(84) Benannte Vertragsstaaten : **BE CH DE FR GB IT LI NL**

(56) Entgegenhaltungen :
**DE A 1 593 857**
**DE A 2 713 777**

(73) Patentinhaber : **BAYER AG**
**Zentralbereich Patente, Marken und Lizenzen**
**D-5090 Leverkusen 1, Bayerwerk (DE)**

(72) Erfinder : **Kranz, Eckart, Dr.**
**Am Acker 9**
**D-5600 Wuppertal 1 (DE)**
Erfinder : **Siegle, Peter, Dr.**
**Am Reinholdsberg 1**
**D-5000 Köln 80 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

# 3,3-Dimethyl-1-phenoxy-butan-2-ole, Verfahren zu ihrer Herstellung sowie ihre Verwendung als Zwischenprodukte

Die vorliegende Erfindung betrifft neue 3,3-Dimethyl-1-phenoxy-butan-2-ole, ein Verfahren zu ihrer Herstellung sowie ihre Verwendung als Zwischenprodukte zur Synthese von bekannten 1-Azolyl-3,3-dimethyl-1-phenoxybutan-2-onen, welche fungizide Eigenschaften aufweisen.

Es sind bereits mehrere Wege zur Herstellung der fungizid wirksamen 1-Azolyl-3,3-dimethyl-1-phenoxy-butan-2-one bekannt geworden (vgl. z.B. DE-PS 22 01 063 (Le A 14 118) und DE-PS 27 13 777 (Le A 17 981) und die allgemeinen Angaben in R. Wegler, « Chemie der Pflanzenschutz- und Schädlingsbekämpfungsmittel », Band 4, Seite 208, Springer-Verlag, Berlin/Heidelberg/New York (1977)). Auf Grund der stetigen Veränderungen auf den Rohstoffmärkten erscheint es jedoch geboten, zwecks besserer Anpassung an die jeweilige Situation nach neuen Vorprodukten und neuen Verfahrenswegen zu suchen.

Es wurden als neue Verbindungen die 3,3-Dimethyl-1-phenoxybutan-2 ole der allgemeinen Formel

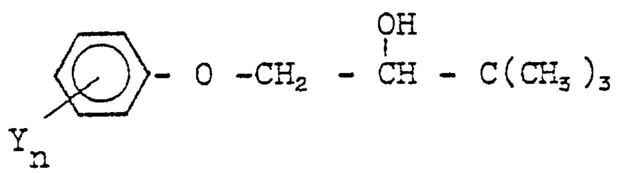

(I)

in welcher

Y für Fluor, Chlor, Brom oder Jod steht, ferner für gegebenenfalls durch Halogen substituiertes Phenyl oder Phenoxy steht, sodann für Nitro und Cyano, ferner für Alkyl, Alkoxy und Alkoxycarbonyl mit jeweils 1 bis 4 Kohlenstoffatomen im Alkylteil steht, sowie für Cycloalkyl mit 5 oder 6 Kohlenstoffatomen steht und

n für 0, 1, 2 oder 3 steht, gefunden.

Weiterhin wurde gefunden, daß man die 3,3-Dimethyl-1-phenoxybutan-2-ole der Formel (I) erhält, wenn man tert.-Butyloxiran der Formel

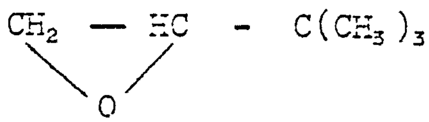

(II)

mit Phenolen der Formel

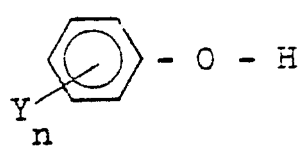

(III)

in welcher

Y und n die oben angegebene Bedeutung haben, in Gegenwart eines Verdünnungsmittels und in Gegenwart einer Base umsetzt.

Die erfindungsgemäßen Verbindungen sind interessante Zwischenprodukte zur Herstellung von bekannten 1-Azolyl-3,3-dimethyl-1-phenoxy-butan-2-onen, welche fungizide Eigenschaften besitzen.

Außerdem zeigen die erfindungsgemäßen Verbindungen wachstumsregulierende Eigenschaften.

Die erfindungsgemäßen Verbindungen sind durch die Formel (I) allgemein definiert.

Als Beispiele seien die Verbindungen der folgenden Tabelle genannt :

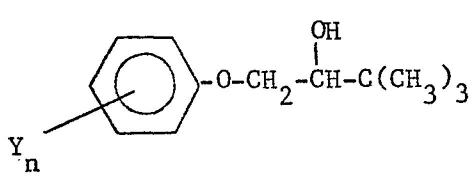

(I)

| $Y_n$ | $Y_n$ |
|---|---|
| 4-Cl | - |
| 4-F | 2-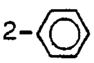 |
| 4-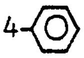 | 4-$C(CH_3)_3$ |
| 4-Br | 2,6-$Cl_2$ |
| 4-$NO_2$ | 2,5-$Cl_2$ |
| 2,4-$Cl_2$ | 2-Cl, 6-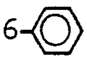 |
| 3-Cl | 2-$CH_3$, 5-$NO_2$ |
| 4-Br, 2-Cl | 4-J |
| 2-$OCH_3$ | 2-$CH_3$, 4-Cl |
| 2,4-$(CH_3)_2$ | 2-F |
| 3,4-$Cl_2$ | 2-$CH_3$ |
| 3-Cl, 4-$NO_2$ | 3-Br |
| 2-$CH_3$, 5-$NO_2$ | 2-$NO_2$ |
| 2-Br, 4-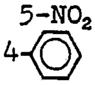 | 4-Cl, 3,5-$(CH_3)_2$ |
| 4-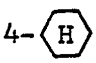 | 4-CO-O-$CH_3$ |
| 2-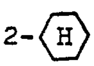 | 4-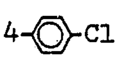-Cl |
| 4-O-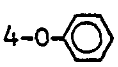 | 2,6-$Cl_2$, 4-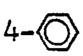-Br |
| 2,6-$Cl_2$, 4-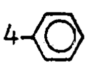 | 2-Cl, 4-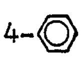-Cl |
| 2,4,6-$Cl_3$ | 4-O-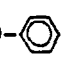-Cl |
| 2-Cl, 4-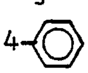 | |

Verwendet man beispielsweise tert.-Butyloxiran und 4-Chlorphenol als Ausgangsstoffe, so kann der Reaktionsablauf durch das folgende Formelschema wiedergegeben werden :

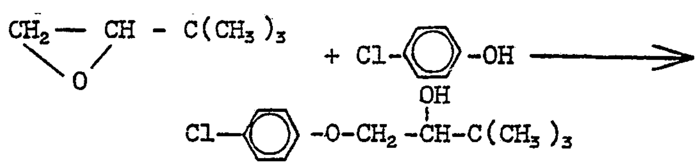

Das bei der Durchführung des erfindungsgemäßen Verfahrens als Ausgangsstoff zu verwendende tert.-Butyloxiran der Formel (II) ist bekannt (vgl. z.B. J. Chem. Soc. *1963,* 1321).

Die außerdem als Ausgangsstoffe zu verwendenden Phenole sind durch die Formel (III) allgemein definiert. In dieser Formel stehen *Y* und der Index *n* vorzugsweise für die Reste, die bei der Beschreibung der erfindungsgemäßen Verbindungen der Formel (I) für diese Substituenten vorzugsweise genannt wurden.

Die Phenole der Formel (III) sind allgemein bekannte Verbindungen der organischen Chemie.

Die erfindungsgemäße Umsetzung wird in Gegenwart einer Base durchgeführt. Als solche können alle üblichen organischen und anorganischen Basen verwendet werden. Hierzu gehören beispielsweise Amine, wie Triethylamin ; Alkalihydroxide, wie Natrium- und Kaliumhydroxid ; oder Alkoholate, wie Natrium- und Kaliummethylat oder -ethylat.

Als Verdünnungsmittel kommen für die erfindungsgemäße Umsetzung Wasser und/oder organische Lösungsmittel infrage. Beispielsweise seien genannt : Ketone, wie Aceton und Methylisobutylketon ; Alkohole, wie Methanol und Ethanol ; Ether, wie Diisopropylether, Tetrahydrofuran und Dioxan ; aromatische Kohlenwasserstoffe, wie Benzol und Toluol ; oder Formamide, wie Dimethylformamid.

Die Reaktionstemperaturen können bei der Durchführung des erfindungsgemäßen Verfahrens in

einem größeren Bereich varriert werden. Im allgemeinen arbeitet man zwischen 0 und 150 °C, vorzugsweise zwischen 20 und 100 °C.

Bei der Durchführung des erfindungsgemäßen Verfahrens setzt man vorzugsweise auf 1 Mol tert.-Butyloxiran der Formel (II) 1-2 Mol, insbesondere 1-1,5 Mol, an Phenol der Formel (III) ein. Die Isolierung der Endprodukte der Formel (I) erfolgt in allgemein üblicher Art und Weise.

Wie schon erwähnt, stellen die neuen 3,3-Dimethyl-1-phenoxy-butan-2-ole der Formel (I) interessante Zwischenprodukte dar. Sie lassen sich leicht in 3,3-Dimethyl-1-phenoxy-butan-2-one der Formel

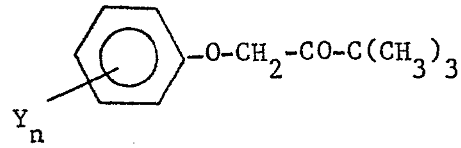

(IV)

in welcher

Y und n die weiter oben angegebene Bedeutung haben, überführen, indem man die Verbindungen der Formel (I) in üblicher Weise oxidiert, wie z.B. mit Luftsauerstoff, Kaliumpermanganat, Braunstein, Chlor oder Kaliumdichromat (vgl. auch die Herstellungsbeispiele) ; oder indem man die Verbindungen der Formel (I) in üblicher Weise dehydriert, wie z.B. mit Edelmetallkatalysatoren, Kupferchromit oder Nitrobenzol.

Die Verbindungen der Formel (IV) können durch weitere Halogenierung, vorzugsweise mit Sulfurylchlorid oder mit Brom, in allgemein üblicher Art und Weise in die entsprechenden 1-Chlor(Brom)-3,3-dimethyl-1-phenoxybutan-2-one überführt werden (vgl. auch DE-OS 24 01 715 [LeA 15 410]), die sich mit 1,2,4-Triazol oder Imidazol glatt zu den 1-Azolyl-3,3-dimethyl-1-phenoxy-butan-2-onen der Formel

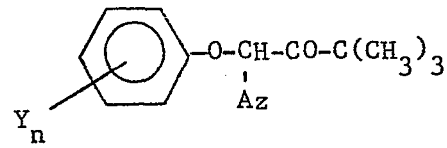

(V)

in welcher

Y und n die weiter oben angegebene Bedeutung haben und

Az für 1,2,4-Triazol-1-yl, 1,2,4-Triazol-4-yl oder Imidazol-1-yl steht, umsetzen lassen (vgl. auch DE-OS 24 01 715 [LeA 15 410]).

Die 1-Azolyl-3,3-dimethyl-1-phenoxy-butan-2-one der Formel (V) weisen starke fungizide Eigenschaften auf (vgl. DE-AS 22 01 063 [LeA 14 118] und DE-OS 23 25 156 [LeA 14 999]).

Wie außerdem ebenfalls schon erwähnt, zeigen die neuen 3,3-Dimethyl-1-phenoxy-butan-2-ole auch eine wachstumsregulierende Wirkung.

Der nachfolgende Vergleichsversuch zeigt z.B. die Wirkung des 1-(4-Chlorphenoxy)-3,3-dimethyl-butan-2-ols gegenüber der unbehandelten Kontrolle bei Zuckerrüben.

Beispiel A

Wuchsbeeinflussung bei Zuckerrüben

Lösungsmittel : 30 Gewichtsteile Dimethylformamid
Emulgator : 1 Gewichtsteil Polyoxyethylen-Sorbitan-Monolaurat.

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit den Wirkstoffzubereitungen besprüht. Nach 14 Tagen wird der Zuwachs der Pflanzen gemessen und die Wuchsbeeinflussung in Prozent des Zuwachses der Kontrollpflanzen berechnet. Es bedeuten 0 % Wuchsbeeinflussung ein Wachstum entsprechend der Kontrollpflanzen. Negative Werte kennzeichnen eine Wuchshemmung, positive Werte eine Wuchsförderung gegenüber den Kontrollpflanzen.

(Siehe die Tabelle A, Seite 5)

TABELLE A

Wuchsbeeinflussung bei Zuckerrüben

| Wirkstoff | Konzentration in ppm | Wuchsbeeinflussung in % |
|---|---|---|
| Kontrolle | — | – 0 |
| 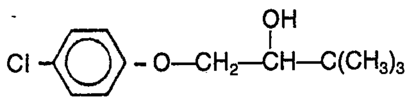 | 500 | + 15* |

* Besonders dicke Blätter

Herstellungsbeispiele

Beispiel 1

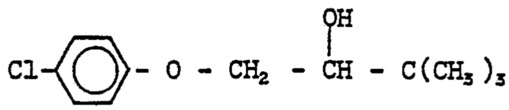

Zu 12,8 g (0,1 Mol) 4-Chlorphenol und 4 g Natriumhydroxid in 20 ml Wasser gibt man 5 g (0,05 Mol) tert.-Butyloxiran. Man läßt das Reaktionsgemisch 24 Stunden bei 60 °C rühren und verdünnt dann mit ca. 100 ml Wasser. Man extrahiert dreimal mit je 50 ml Methylenchlorid. Die vereinigten organischen Phasen werden zwecks Entfernung nicht umgesetzten Phenols mit verdünnter waßriger Natronlauge gewaschen, über Natriumsulfat getrocknet und eingeengt. Der Rückstand wird im Vakuum destilliert. Man erhält 5,9 g (52 % der Theorie) 1-(4-Chlorphenoxy)-3,3-dimethyl-butan-2-ol vom Siedepunkt 105 °C/0,5 mm Hg.

Folgeprodukte

a)

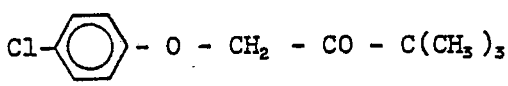

11,4 g (0,05 Mol) 1-(4-Chlorphenoxy)-3,3-dimethyl-butan-2-ol werden bei 30 °C zu einer Mischung von 14,7 g (0,05 Mol) Kaliumdichromat in 12,25 g Schwefelsäure und 73,5 g Wasser gegeben, wobei die Temperatur auf ca. 45 °C ansteigt. Man läßt über Nacht bei Raumtemperatur rühren und extrahiert dann dreimal mit Ether. Die vereinigten organischen Phasen werden mit Natriumhydrogencarbonat und Wasser gewaschen, über Natriumsulfat getrocknet und eingeengt. Man erhält 7,8 g (69 % der Theorie) 1-(4-Chlorphenoxy)-3,3-dimethyl-butan-2-on vom Schmelzpunkt 58 °C.

b)

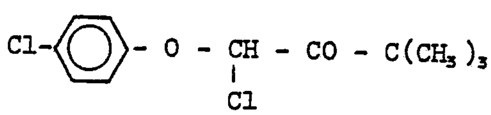

22,7 g (0,1 Mol) 1-(4-Chlorphenoxy)-3,3-dimethyl-butan-2-on in 100 ml Tetrachlorkohlenstoff werden auf 60 °C erwärmt. Zu dieser Lösung werden 16,2 g (0,12 Mol) Sulfurylchlorid ohne weitere Erwärmung so zugetropft, daß eine stetige Gasentwicklung erfolgt. Nach beendeter Zugabe läßt man unter Rückfluß rühren und engt dann durch Abdestillieren des Lösungsmittels im Vakuum ein. Man erhält in quantitativer Ausbeute 1-(4-Chlorphenoxy)-1-chlor-3,3-dimethyl-butan-2-on, das ohne weitere Reinigung direkt weiter umgesetzt wird.

c)

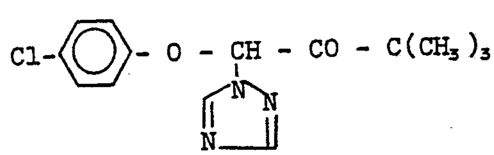

41,8 g (0,66 Mol) 1,2,4-Triazol werden in 300 ml Aceton gelöst. Hierzu werden 93,4 g (0,72 Mol) wasserfreies Kaliumcarbonat gegeben, die Suspension zum Sieden erhitzt und eine Lösung von 156,5 g (0,6 Mol) 1-(4-Chlorphenoxy)-1-chlor-3,3-dimethyl-butan-2-on in 150 ml Aceton so zugetropft, daß die Mischung ohne Beheizung am Rückfluß siedet. Nach beendeter Zugabe wird zur Vervollständigung der Reaktion 15 Stunden unter Rückfluß erhitzt ; anschließend wird der erhaltene Niederschlag abfiltriert, mit Aceton gewaschen und verworfen. Das Filtrat wird im Wasserstrahlvakuum vom Lösungsmittel befreit, der Rückstand in 300 ml Toluol aufgenommen und einmal mit einer Lösung von 10 g 37 %-iger Salzsäure in 200 ml Wasser gewaschen. Die wässrige Phase wird abgetrennt und verworfen ; die organische Phase wird mit 500 ml Wasser gewaschen und — nach Zugabe von weiteren 400 ml Toluol — mit einer Lösung von 14,5 g Natriumhydroxid in 350 ml Wasser 6 Stunden bei Raumtemperatur gerührt. Danach wird die organische Phase abgetrennt, mit Wasser neutral gewaschen und im Wasserstrahlvakuum vom Lösungsmittel befreit. Man erhält 153,5 g (87 % der Theorie) 1-(4-Chlorphenoxy)-3,3-dimethyl-1-(1,2,4-triazol-1-yl)-butan-2-on vom Schmelzpunkt 75-76 °C.

Die Verbindung und ihre fungizide Wirkung sind bekannt (vgl. DE-PS 22 01 063 (Le A 14 118) bzw. US-PS 3 912 752).

**Ansprüche**

1. 3,3-Dimethyl-1-phenoxy-butan-2-ole der allgemeinen Formel

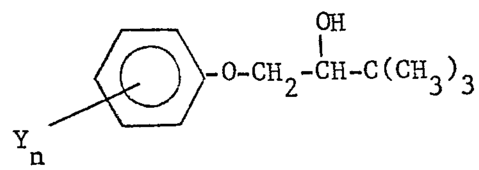

(I)

in welcher

Y für Fluor, Chlor, Brom oder Jod steht, ferner für gegebenenfalls durch Halogen substituiertes Phenyl oder Phenoxy steht, sodann für Nitro und Cyano, ferner für Alkyl, Alkoxy und Alkoxycarbonyl mit jeweils 1 bis 4 Kohlenstoffatomen im Alkylteil steht, sowie für Cycloalkyl mit 5 oder 6 Kohlenstoffatomen steht und

n für 0, 1, 2 oder 3 steht.

2. Verfahren zur Herstellung von 3,3-Dimethyl-1-phenoxy-butan-2-olen der Formel I, dadurch gekennzeichnet, daß man tert.-Butyloxiran der Formel

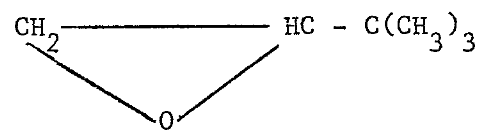

(II)

mit Phenolen der Formel

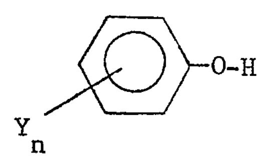

(III)

in welcher

Y und n die in Anspruch 1 angegebene Bedeutung haben, in Gegenwart eines Verdünnungsmittels und in Gegenwart einer Base umsetzt.

3. Verwendung von 3,3-Dimethyl-1-phenoxy-butan-2-olen der Formel I als Zwischenprodukte zur Herstellung von 1-Azolyl-3,3-dimethyl-1-phenoxy-butan-2-onen, dadurch gekennzeichnet, daß man die 3,3-Dimethyl-1-phenoxy-butan-2-ole der Formel I in üblicher Weise oxidiert, und die dabei erhaltenen 3,3-Dimethyl-1-phenoxy-butan-2-one der allgemeinen Formel

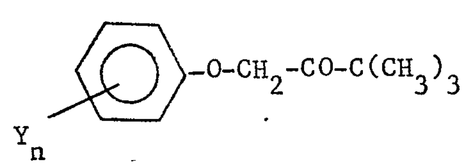

(IV)

in welcher

Y und n die in Anspruch 1 angegebene Bedeutung haben, durch Halogenierung in üblicher Weise in die entsprechenden 1-Halogen-3,3-dimethyl-1-phenoxy-butan-2-one überführt und diese dann mit Azolen zu den 1-Azolyl-3,3-dimethyl-1-phenoxy-butan-2-onen der Formel

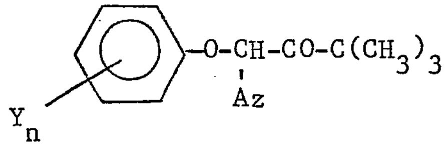

(V)

in welcher

Y und n die in Anspruch 1 angegebene Bedeutung haben und

Az für 1,2,4-Triazol-1-yl, 1,2,4-Triazol-4-yl oder Imidazol-1-yl steht, überführt.

**Claims**

1. 3,3-Dimethyl-1-phenoxy-butan-2-ols of the general formula

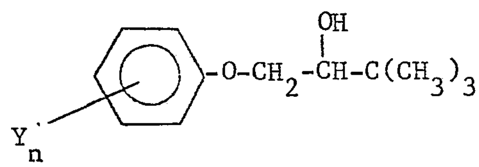

(I)

in which

Y represents fluorine, chlorine, bromine or iodine, furthermore phenyl or phenoxy optionally substituted by halogen, then nitro and cyano, furthermore alkyl, alkoxy and alkoxycarbonyl with in each case 1 to 4 carbon atoms in the alkyl part, as well as cycloalkyl with 5 or 6 carbon atoms and

n represents 0, 1, 2 or 3.

2. Process for the preparation of 3,3-dimethyl-1-phenoxy-butan-2-ols of the formula I, characterised in that tert.-butyloxirane of the formula

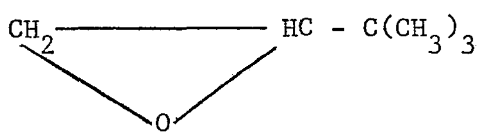

(II)

is reacted with phenols of the formula

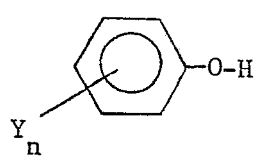

(III)

in which

Y and n have the meaning given in Claim 1, in the presence of a diluent and in the presence of a base.

3. Use of 3,3-dimethyl-1-phenoxy-butan-2-ols of the formula I as intermediate products for the preparation of 1-azolyl-3,3-dimethyl-1-phenoxy-butan-2-ones, characterised in that the 3,3-dimethyl-1-phenoxy-butan-2-ols of the formula I are oxidised in the customary manner and the 3,3-dimethyl-1-phenoxy-butan-2-ones thereby obtained, of the general formula

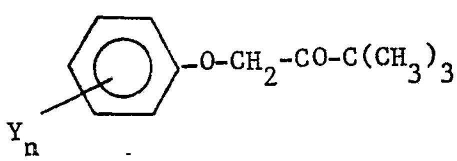

(IV)

in which

Y and n have the meaning given in Claim 1, are converted into the corresponding 1-halogen-3,3-dimethyl-1-phenoxy-butan-2-ones by halogenation in the customary manner and these products are then converted into the 1-azolyl-3,3-dimethyl-1-phenoxy-butan-2-ones of the formula

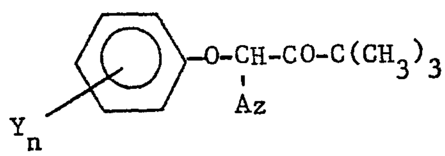

(V)

in which

Y and n have the meaning given in Claim 1 and

Az represents 1,2,4-triazol-1-yl, 1,2,4-triazol-4-yl or imidazol-1-yl, with azoles.

**Revendications**

1. 3,3-diméthyl-1-phénoxy-butane-2-ols de formule générale

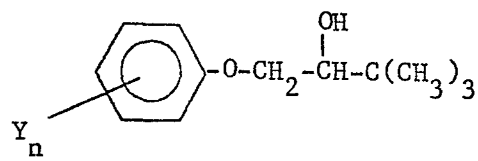

(I)

dans laquelle

Y représente le fluor, le chlore, le brome ou l'iode, en outre un reste phényle ou phénoxy éventuellement substitué par un halogène, ensuite un groupe nitro et un groupe cyano, en outre un groupe alkyle, alkoxy et alcoxycarbonyle ayant chaque fois 1 à 4 atomes de carbone dans le reste alkyle, ainsi qu'un reste cycloalkyle ayant 5 ou 6 atomes de carbone, et

n est égal 0, 1, 2 ou 3.

2. Procédé pour la préparation de 3,3-diméthyl-1-phénoxy-butane-2-ols de formule I, caractérisé en ce que l'on fait réagir le tert-butyloxiranne de formule

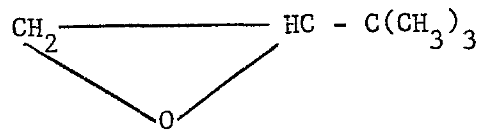

(II)

avec des phénols de formule

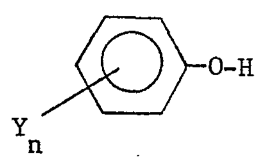

(III)

dans laquelle

Y et n ont la signification déjà indiquée à la revendication 1, en présence d'un agent diluant et en présence d'une base.

3. Utilisation de 3,3-diméthyl-1-phénoxy-butane-2-ols de formule I comme produits intermédiaires pour la préparation de 1-azolyl-3,3-diméthyl-1-phénoxy-butane-2-ones, caractérisée en ce que l'on oxyde de la manière habituelle les 3,3-diméthyl-1-phénoxy-butane-2-ols de formule I et on transforme les 3,3-diméthyl-1-phénoxy-butane-2-ones ainsi obtenus de formule générale

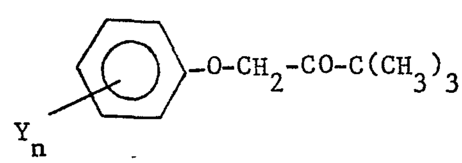

(IV)

dans laquelle

Y et n ont la signification indiquée à la revendication 1, en 1-halogéno-3,3-diméthyl-1-phénoxy-

butane-2-ones correspondantes par halogénation de la manière habituelle et on transforme celles-ci avec des azoles en les 1-azolyl-3,3-diméthyl-1-phénoxy-butane-2-ones de formule

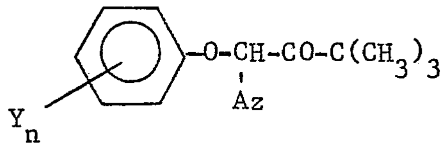

(V)

dans laquelle

Y et n ont la signification indiquée à la revendication 1 et

Az représente un reste 1,2,4-triazole-1-yle, 1,2,4-triazole-4-yle ou imidazole-1-yle.